Europäisches Patentamt

European Patent Office

Office européen des brevets

(19)

(11) Publication number: **0 000 677**
**B1**

(12) # EUROPEAN PATENT SPECIFICATION

(45) Date of publication of patent specification: **05.08.81**

(21) Application number: **78400058.0**

(22) Date of filing: **12.07.78**

(51) Int. Cl.³: **C 12 N 7/08,**
**A 61 K 39/155**

(54) Parainfluenza virus vaccine and its preparation.

(30) Priority: **22.07.77 US 817955**

(43) Date of publication of application:
**07.02.79 Bulletin 79/3**

(45) Publication of the grant of the European patent:
**05.08.81 Bulletin 81/31**

(84) Designated Contracting States:
**BE CH DE FR GB LU NL SE**

(56) References cited:
**JP - A - 74 040 926**

(73) Proprietor: **MERCK & CO. INC.**
**126, East Lincoln Avenue P.O. Box 2000**
**Rahway, New Jersey 07065 (US)**

(72) Inventor: **Buynak, Eugene Bernard**
**Gwynedd View Road**
**North Wales Pennsylvania (US)**
Inventor: **Hilleman, Maurice Ralph**
**4107 Fields Drive**
**Lafayette Hill Pennsylvania (US)**

(74) Representative: **Corre, Jacques Denis Paul et al,**
**Cabinet Regimbeau 26, Avenue Kléber**
**F-75116 Paris (FR)**

Courier Press, Leamington Spa, England.

Parainfluenza virus vaccine and its preparation

In general terms, the invention is concerned with the adaptation and propagation of parainfluenza type 3 in tissue cultures prepared from embryonated hens' eggs. More particularly, this invention is directed to the development of live virus vaccines against the parainfluenza group of agents following serial passage in chick embryo tissue culture. This procedure involves the steps of

A) the isolation of the virulent viruses in any of a variety of cells in culture, and its adaptation to chick embryo tissue culture;

B) the development of the attenuated viruses by a plurality of serial passages in chick embryo tissue culture; and

C) the preparation of the vaccines from these attenuated live viruses.

These steps will be separately explained.

A. *Isolation and adaptation of virulent virus*

Isolation and adaptation of parainfluenza virus can be accomplished in chick embryo tissue culture using virus previously propagated in another kind of cell culture, such as monkey kidney or a combination of monkey kidney and embryonated hens' eggs. Isolation in the above-mentioned cell cultures can be from clinical material (e.g. throat swab). Incubation of infected cultures can be carried out at any temperature between about 30° and about 38°C, preferably at 30—34°C (optimal 32°C) or at 35 to 38°C (optimal 36°C). From about 3 to about 20 serial passages may be employed to attenuate the virus. Parainfluenza virus type 3 is isolated and adapted by at least 1 passage in monkey kidney cell culture.

B. *Development of attenuated live parainfluenza virus vaccine*

The virus which has been established in A to be parainfluenza virus is added to glass bottles containing chick embryo tissue cultures prepared from minced and trypsinized approximately ten-day-old chick embryos. The culture medium may be any of those which support cell growth and this may, for example, be the known medium 199 to which calf serum has been added. After the addition of the virus, the infected cell cultures are incubated in successive passages at 30—38°C and preferably at 30—34°C (optimal 32°C) and 35—38°C (optimal 36°C). During these passages the virus is replicated in large amount and becomes attenuated.

The above serial passages are performed using undiluted or diluted inoculum and multiple harvests are collected at various intervals. Infectivity titrations are performed in grivet monkey kidney tissue cultures.

C. *Preparation of vaccine from attenuated virus*

The parainfluenza virus harvested after this repeated serial passage is found to be non-pathogenic for monkeys and rodents, to cause little or no clinical reactions in human recipients, and to evoke a satisfactory level of neutralizing antibody. The virus infectivity is stabilized by a suitable stabilizer such as sucrose, phosphate, glutamine, human albumin, or mixtures thereof. After titration to establish its potency, the virus pool is subdivided and filled into appropriate vials for use. The product can be stored frozen or preferably dried from the frozen state and kept free of moisture.

The following example illustrates the present invention without, however, limiting the same thereto.

Example

The inoculum is parainfluenza virus type 3 which is obtained as described in A above after 2 passages in grivet monkey kidney cell culture and 9 passages in chick embryo tissue culture. Nine to eleven-day-old chick embryos, after removal of the head and extremities, are finely minced under aseptic conditions and the minced tissue washed in several changes of Hank's Balanced Salt Solution (BSS). The washed tissue is trypsinized at 36°C using 0.25% trypsin (Difco 1:250) in tris saline buffer for approximately two hours. The trypsin-cell suspension is harvested through two thicknesses of sterile cheese cloth and centrifuged at 1500 rpm for five minutes. Packed cells are resuspended in growth medium for counting. Growth medium consists of medium 199 (Morgan, J. F., Morton, H. J., and Parker, R. C., Proc. Soc. Exp. Biol. and Med., *73*: 1—8, 1950) containing 2% unheated fetal calf serum and 50 mcg/ml neomycin. Bottle cultures are planted at a concentration of 700,000 viable cells per milliliter. Following incubation at 36°C for 48 to 72 hours, bottle cultures can be used for serial passage or vaccine preparation.

Chick embryo tissue cultures are prepared in glass bottles using medium 199 containing 2% unheated fetal calf serum as growth medium. Three to four days post-planting, the growth medium is decanted and the cultures inoculated with 5.0—10.0 ml of undiluted or diluted seed virus per bottle. Following an adsorption period of one hour at 30—34°C, 70 milliliters of medium 199 containing 2% a gamma calf serum is added to each bottle, and re-incubated at 30—34°C. Three to four days post-seeding, the bottle cultures are washed four times with Hanks' BSS, 100 milliliters per wash. Following the washing procedure, 100 milliliters of medium 199 containing a suitable viral stabilizer is added to each bottle and the cultures incubated at 30—34°C. Neomycin at a concentration of 50 mcg/ml is incorporated in the growth and maintenance medium. Multiple harvests are collected at 2—4 day intervals and the bottle cultures are refed with fresh maintenance medium containing stabilizer. Infectivity titrations of each harvest are performed in grivet monkey kidney tissue cultures. Each harvest is collected aseptically into a sterile container, samples removed for microbial sterility testing and the remainder is shell-frozen in a dry ice-alcohol bath. The virus-containing fluids are stored at 70°C in an electrically operated freezing unit prior to selection of a harvest or harvests for preparation of the vaccine.

Appropriate harvest or harvests are selected following completion of infectivity titrations. The selected material is removed from the freezer and thawed. A sample is removed for control and safety testing. The remaining fluid is clarified and a sample removed for monkey safety testing. Appropriate additional stabilizer, as mentioned above, is added to the remaining fluid. The fluids are distributed into individual vials and lyophilized. Following the lyophilization cycle the vials are capped, sealed and retained for reconstitution as a vaccine by the addition of sterile water (Water for Injection, U.S.P.).

The potency of the product is based on infectivity titrations in grivet monkey kidney cell cultures.

*Tests in man*

Clinical studies of parenterally administered parainfluenza virus type 3 (monovalent vaccine) were conducted in children. Good antibody responses were observed with little, if any, untoward clinical reactions.

## Claims

1. A process of preparing a live, immunogenic parainfluenza virus type 3, characterized in that it comprises isolating and adapting the virus by at least 1 passage in monkey kidney cell culture and serially passaging the virus from about 3 to about 20 times in chick embryo cell culture.

2. A process according to claim 1, characterized in that the isolation and adaptation is carried out at a temperature of from about 30°C to about 38°C.

3. A parainfluenza vaccine composition, characterized in that it comprises a virus obtained by the process according to claims 1 or 2 together with a viral stabilizer.

4. A parainfluenza vaccine composition according to claim 3, characterized in that said stabilizer comprises sucrose, phosphate, glutamin or human albumin, or mixtures thereof.

5. A parainfluenza vaccine composition, according to claims 3 or 4, characterized in that it is in frozen state.

6. A parainfluenza vaccine composition

according to claims 3 or 4, characterized in that it is in lyophilized state.

## Patentansprüche

1. Verfahren zur Herstellung eines lebenden, immunogenen Parainfluenza-Virus-Typ 3, dadurch gekennzeichnet, dass man das Virus durch mindestens 1 Passage in Affennieren-Zellkultur isoliert und adaptiert und das Virus etwa 3- bis 20-mal einer Serienpassage in Hühnerembryo-Zellkultur unterwirft.

2. Verfahren nach Anspruch 1, dadurch gekennzeichnet, dass man die Isolation und Adaption bei einer Temperatur von etwa 30°C bis etwa 38°C durchführt.

3. Parainfluenza-Vaccinezusammensetzung, dadurch gekennzeichnet, dass sie ein gemäss Anspruch 1 oder 2 erhaltenes Virus zusammen mit einem Virusstabilisator enthält.

4. Parainfluenza-Vaccinezusammensetzung nach Anspruch 3, dadurch gekennzeichnet, das der Stabilisator Saccharose, Phosphat, Glutamin oder Humanalbumin oder Gemische davon enthält.

5. Parainfluenza-Vaccinezusammensetzung nach Anspruch 3 oder 4, dadurch gekennzeichnet, dass sie in gefrorenem Zustand vorliegt.

6. Parainfluenza-Vaccinezusammensetzung nach Anspruch 3 oder 4, dadurch gekennzeichnet, dass sie in lyophilisiertem Zustand vorliegt.

## Revendications

1. Procédé de préparation d'un virus parainfluenza immunogène type 3 vivant, caractérise en ce qu'il implique d'isoler et d'adapter le virus par au moins un passage dans une culture cellulaire de rein de singe et une série de passages du virus, d'environ 3 à environ 20 fois, dans une culture cellulaire d'embryon de poulet.

2. Procédé selon la revendication 1, caractérisé en ce que l'isolement et l'adaptation s'effectuent à une température d'environ 30°C à environ 38°C.

3. Composition de vaccin contre le virus paràinfluenza, caractérisée en ce qu'elle comprend un virus obtenu par le procédé selon l'une des revendications 1 ou 2 avec un stabilisateur viral.

4. Composition de vaccin selon la revendication 3, caractérisée en ce que ledit stabilisateur comprend du saccharose, du phosphate, de la glutamine ou de l'albumine humaine, ou leurs mélanges.

5. Composition de vaccin selon l'une des revendications 3 ou 4, caractérisée en ce qu'elle est à l'état congelé.

6. Composition de vaccin selon l'une des revendications 3 ou 4, caractérisée en ce qu'elle est à l'état lyophilisé.